# EUROPEAN PATENT APPLICATION

(11) **EP 3 647 784 A1**
(43) Date of publication of application: **06.05.2020**
(21) Application number: 18806532.0
(22) Date of filing: 24.05.2018
(51) Int. Cl.: G01N 33/53, G01N 33/58, C12N 5/071

(54) **ISOLATION OF CELLS OF EPITHELIAL ORIGIN CIRCULATING IN PERIPHERAL BLOOD**

(30) Priority: 24.05.2017 ES 201730724
(71) Applicant: Universidad de Granada, E-18071 Granada (ES); Servicio Andaluz de Salud, 41071 Sevilla (ES)
(72) Inventor: ROMERO PALACIOS, Pedro Jos, 18071 Granada (ES); D AZ MOCH N, Juan Jos, 18071 Granada (ES); LORENTE ACOSTA, Jos Antonio, 18071 Granada (ES); DE MIGUEL P REZ, Diego, 18071 Granada (ES); SERRANO FERN NDEZ, Mar a Jos, 18012 Granada (ES); ALC ZAR NAVARRETE, Bernardino, 18012 Granada (ES)
(74) Representative: Elzaburu S.L.P.
(86) International application number: PCT/ES2018/070377
(87) International publication number: WO 2018/215686

(57) **Abstract**

The present invention provides a method for identifying epithelial cells circulating in peripheral blood, which allows individuals who suffer from a disease that presents with epithelial cell destruction to be discriminated from those who do not. The invention also relates to a kit or device for carrying out the methods of the invention.

## Description

### FIELD OF THE INVENTION

The present invention is included within the field of medicine, and more specifically, it relates to a method for identifying epithelial cells circulating in peripheral blood which allows individuals who suffer from a disease that presents with epithelial cell destruction to be discriminated from those who do not, and the kit or device for carrying out the methods of the invention.

### STATE OF THE ART

The epithelium is a tissue composed of closely linked cells, with little intercellular substance. The epithelial cells are derived from two of the embryonic germ layer: the ectoderm and the endoderm, and structurally compose the majority of organs.

From the endoderm is derived the epithelium of the epidermis and the cornea. Glandular annexes such as the sweat glands, sebaceous glands and mammary glands.

From the endoderm is derived the tissue covering the digestive tube, also of endodermal origin. All the glands that integrate the digestive apparatus, such as the liver, pancreas and gastric and intestinal glands.

The mesoderm gives origin to the liver, and male and female genital apparatus.

A fundamental property of epithelial cells is their tendency to maintain a very large contact between one another, forming coherent layers that coat surfaces and cover cavities.

The respiratory epithelium has a twofold origin: the epithelium of the larynx, trachea, bronchial tubes and pulmonary alveola has an endodermal origin, whilst the cartilaginous and muscular structures and the vascular system have mesodermal origin.

The epithelial cells, especially those that cover the external surface of the body and the intestine, are constantly subjected to mechanical traumas and traumas of other types. In physiological conditions, cells die constantly in these epitheliums which are then released. In the gastrointestinal tract, the cells suffer continuous exfoliation, in the points of the hairs.

In contrast, in the airways and especially in the majority of the glands, the degeneration of the epithelium is rare and, therefore, the life span of the cells is quite large.

The physiological loss of the cells in the epithelium is compensated with the corresponding regeneration, which in vertebrates is produced by means of the mitotic proliferation of relatively undifferentiated cell elements.

In lung tissue, the alveolar macrophages are those in charge of phagocytizing and eliminating degraded lung tissue, including Type I and Type II pneumocytes and surfactant.

When, due to their nature, the particles cannot be digested by the macrophage, it migrates with them in its phagosome until the start of the mucociliary transport, when they are mobilized until the oropharynx and deglutinated or eliminated with the sputum.

COPD (Chronic Obstructive Pulmonary Disease) is one of the respiratory diseases with greatest prevalence and morbimortality on a global level. It is the third cause of death just behind cardiovascular diseases and cancer and it affects close to 10% of the adult population in developed countries.

Furthermore, a high proportion of patients are not diagnosed or do not receive suitable treatment for their disease. More of 70% of patients are unaware that they suffer from it, with many of them remaining undiagnosed even until very advanced phases of the disease.

COPD is characterized by a progressive loss of lung function fundamentally as a consequence of the exposure to tobacco smoke, which is the physiological translation of inflammatory and immune processes which lead to the destruction of alveolar units, loss of the small-calibre airways and the peribronchial fibrosis characteristic of the disease. As a consequence of these processes, remains of the degradation of the pulmonary parenchyma are released into the general circulation, as elements of the extracellular matrix.

The loss of lung function produced in COPD may be different among patients, and it is possible that the processes that alter the pulmonary maturation in childhood may significantly predispose towards the development of the disease. However, detecting the patients that lost lung function in an accelerated manner is not feasible and they do not have specific biomarkers of the pathological pulmonary processes.

In recent years we have seen the development of techniques which allow the identification in peripheral blood of cells from human tissues, grouped under the term liquid biopsy, and which have demonstrated a diagnostic usefulness in the field of oncology. Especially interesting have been the advances that the liquid biopsies have provided in the monitoring of patients with lung cancer, adding prognostic information and allowing a personalization of treatment for these patients. Knowing the mechanisms involved in the pathogeny of COPD, it is possible that alveolar cells or their remains may be discharged into the blood stream, and that they can be identified in the tumor cells.

The current diagnosis of COPD is fundamentally based on clinical manifestations, - cough, expectoration, dyspnea-, and lung function tests, mainly spirometry -FEV1 <0.7 after bronchodilators-. In both cases, it relates to epiphenomena, clinical symptoms and bronchial obstruction, which occur when the physiopathological alterations of the disease are already installed, with there not being any markers that allow an early diagnosis thereof, until it becomes irreversible, as occurs in the majority of diagnosed cases of COPD. The average annual direct cost of a patient with COPD in Spain has been quantified between €910 and €2060. Having a diagnostic method that allows the early identification of those individuals who suffer from the disease, or who have a high risk of developing it, improving the sensitivity and specificity of the current proceedings, would decrease the economic costs and improve the prognosis of said patents.

### BRIEF DESCRIPTION OF THE INVENTION

The authors of the present invention have determined the sensitivity and specificity *in vitro* of the first technique for detecting circulating pulmonary cells (CPCs) and they have then demonstrated that it is possible to isolate CPCs in peripheral blood of patients with COPD.

Although, to date, no references have been found that demonstrate the presence of well-differentiated epithelial cells in the bloodstream, their presence would be indicative of the existence of intense processes of tissue aggression, which exceed the capacity of the macrophages to eliminate the "waste" material, and other physiological mechanisms and this is an especially significant fact in epithelial tissues such as lung and glandular structures.

Therefore, the technique disclosed in the present invention, which, for the first time, manages to detect the presence of well-differentiated epithelial cells in peripheral blood, would serve for the diagnosis of individuals who suffer from COPD, and for any disease that presents with tissue destruction and release of epithelial cells.

### BRIEF DESCRIPTION OF THE FIGURES

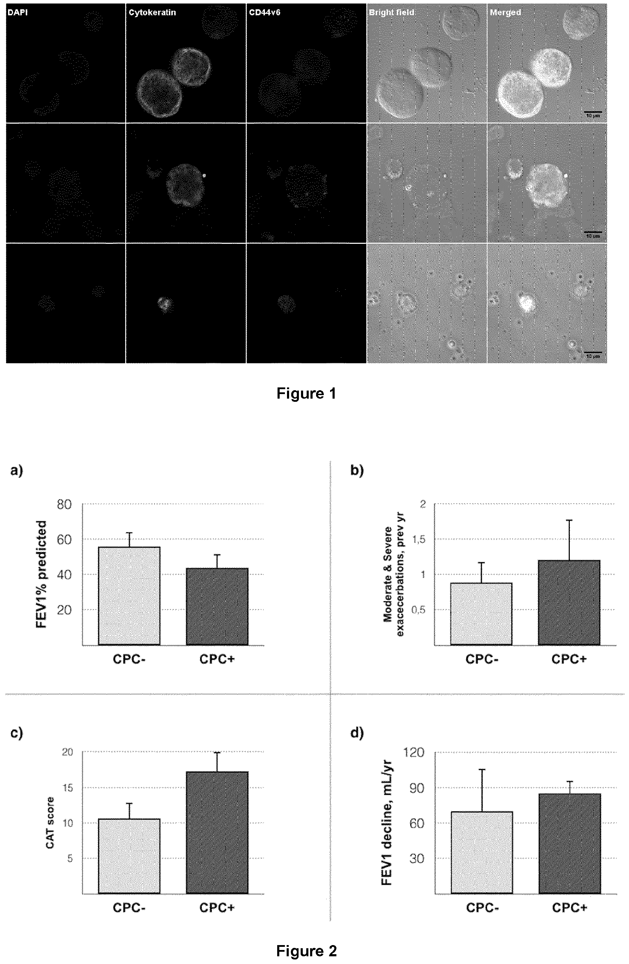

**Figure 1****.** Immunofluorescent characterization and cytomorphology of H820 cells 'spiked' in blood and CPC of patients with COPD. The columns show signal for (blue) 4', 6-Diamidino-2-Phenylindole, Dilactate (DAPI) for the staining of the nucleic acid (nuclear), expression of cytokeratin-FITC (green), purple (reddish purple) expression of CD44v6-Alexa Fluor® 633, (greys) display of bright field and merged channels. The first row represents isolated H820 lung cancer cells from enrichment experiments, with positive expression of cytokeratin and CD44v6. The second and third rows represent CPC isolated from patients with COPD with positive signal for cytokeratin and CD44v6.
**Figure 2****.-** Characteristics of COPD of the participants in the study based on the presence of CPC isolated from blood samples. a) Lung function values (expressed as % of the prediction). b) Annualized rate of exacerbation in the previous year. c) CAT scores (COPD assessment test). d) Decline in lung function (expressed as annualized FEV1 decline in ml / year). The bar represents the mean and SEM values. CPC +: isolated CPC; CPC -: the CPC are not isolated

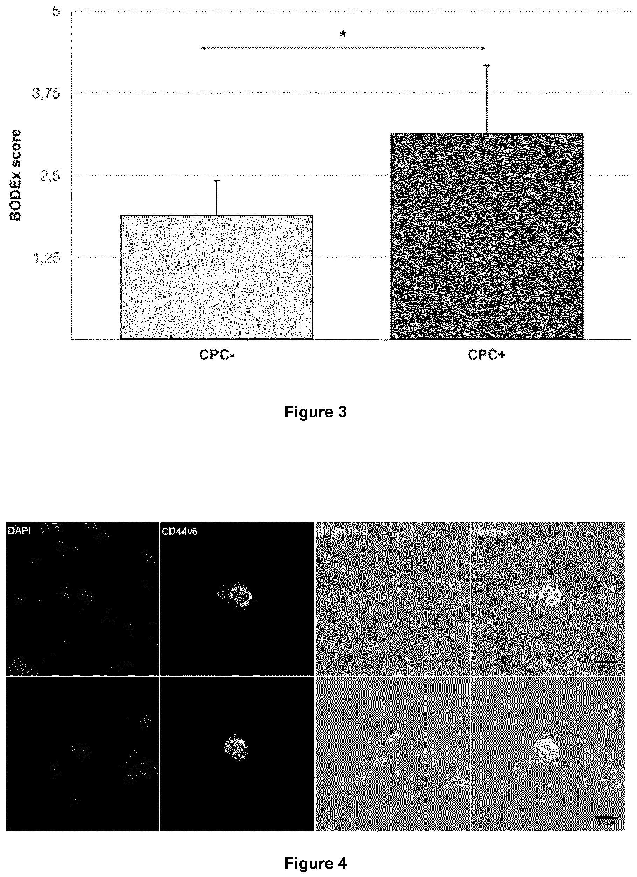

**Figure 3****.**- Differences in the BODEx index depending on the presence of isolated CPC in blood samples. The bars represent Mean and SEM. * = p <0.05. CPC +: isolated CPC; CPC-: the CPC are not isolated
**Figure 4**.-Immunofluorescent and histomorphological characterization of pneumocytes from non-tumor lung tissues. The columns show signal for (blue) 4 ', 6-Diamidino-2-Phenylindole, Dilactate (DAPI) or nucleic acid staining (nuclear), expression (green) CD44v6-FITC, display of bright field (greys) and merged channels. The rows represent lung tissue of patient with pneumothorax.

### DETAILED DESCRIPTION OF THE INVENTION

The degraded epithelial cells have physiological mechanisms of elimination, mainly via phagocytizing by macrophages. Thus, in the case of lung tissue, the alveolar macrophages are those responsible for phagocytizing and eliminating degraded pulmonary materials, including Type I and Type II pneumocytes and surfactant.

When, due to their nature, the particles cannot be digested by the macrophage, it migrates with them in its phagosome until the start of the mucociliary transport, when they are mobilized until the oropharynx and deglutinated or eliminated with the sputum.

Thus, the diseases that may give rise to the presence of circulating epithelial cells would be those that impact the liver, pancreas, intestine, gastric and intestinal glands and genital organs.

The present invention discloses a method to detect epithelial cells which, surprisingly, can be detected in the blood stream.

Specifically, the method of the present invention discloses an automated method of isolation, detection and phenotypical and genetic characterization of cells from epithelial tissues and which may be detected in the blood. The detection of said cells in peripheral blood is determined by the existence of a physiopathological process which gives rise to destructuring phenomena and tissue destruction, such as, for example but without being limited to, the case of Chronic Obstructive Pulmonary Disease -COPD -, or the existence of tumor disease. Thus, in the first case we could speak of Circulating Pulmonary Cells (CPCs) and in the second case of Circulating Tumor Cells (CTCs).

The capacity of the tumor cells to produce metastasis remotely is widely known, and numerous clinical studies and basic research have been centred on in their detection and characterization. Nevertheless, to date, no circulating epithelial cells have been detected in the bloodstream. The method of the present invention centres its clinical application on the possibility of detecting non-tumor circulating epithelial cells, as specific biomarker of tissue damage. This technique could be applied to the detection of any epithelial cell, using the specific markers that identify them. Furthermore, it also allows the use of intracytoplasmic and nuclear markers for automated cell isolation.

### METHODS OF THE INVENTION

Therefore, a **first aspect** of the invention relates to a method for identifying epithelial cells circulating in peripheral blood, hereinafter first method of the invention, comprising the steps of incubating the sample of peripheral blood with the intracytoplasmic, nuclear and/or surface markers in accordance with the circulating epithelial cell, and of their tissue origin to detect, and identify the cells by means of immunocytochemical, molecular and/or cytogenetic techniques.

Preferably, the first method of the invention comprises a prior step of subjecting the sample of peripheral blood of an individual to a gradient density separation.

In another preferred embodiment, a step is performed after the gradient density separation consisting of collecting the interphase and washing it with saline buffer before incubating the sample with the markers.

The cells identified by the first method of the invention are, in principle, preferably non-tumor cells. Even more preferably, they are non-tumor cells from body tissues affected by any process of tissue aggression. In another more preferred embodiment, they are epithelial cells from the lungs, the liver, the pancreas and cells from the gastric and intestinal glands, the liver, genital organs, or any combinations thereof.

In another preferred embodiment, the intracytoplasmic markers are selected from the list consisting of: Cytokeratins, Vimentin, SP1 (Surfactant A and B antigen), or any combinations thereof.

In another preferred embodiment, the nuclear markers are transcription factors, and preferably are selected from Snail, Slug, SOX2, or any combinations thereof.

In another preferred embodiment, the surface markers are selected from the list consisting of N-cadherin, EpCam, CD44v6, AXL, EGFR, EMA, MUCIN, CD133, Sca1, In Asialoglycoprotein (ASGPR) or any combinations thereof.

More preferably, the markers are of CPCs, and are selected from the list consisting of: Cytokeratins, CD44V6, SP1, Sca1 and SOX2. In an even more preferred embodiment, the circulating epithelial cell which is detected with the first method of the invention is a circulating pulmonary cell (CPC).

In another more preferred embodiment, the markers are of liver cells, and are selected from the list consisting of: sialoglycoprotein, mir122, CXCR4 (CD184), CD90, ALDH1.

In another more preferred embodiment, the markers are of pancreas cells, and are selected from the list consisting of: KRAS, TP53,CDKN2A/p16, MAD4/DPC4, miR-7, miR-375, miR-16, miR-196a miR-16, miR-196, miR-27a-3p miR-145, miR-150, miR-223, miR-636, miR-21, miR-196a, miR-196b, miR-18a, miR-223 and miR-221.

In another more preferred embodiment, the markers are of cells from gastric and intestinal glands (separate if necessary), and are selected from the list consisting of: miR-557; SPI1, NFIC, SPIB, THAP1, urokinase- type tissue plasminogen activator (upaR).

In another more preferred embodiment, the markers are of kidney cells, and are selected from the list consisting of: CK7, AMACR, CA IX, TFE3, upaR.

In another more preferred embodiment, the markers are of cells from the genital organs, and are selected from the list consisting of: BRAC1/2, IPK3, iR-132, miR-26a, miR-145, EMG1 and SEMG2, FOLH1, TGM4, TKTL1, LDHC and PGK2.

In another more preferred embodiment, the markers are of cells from the central nervous system, and are selected from the list consisting of: Nestin, SOX10, Notch1, HES1, HES2, Occludin, PAX6, HES5, GABA_{B} receptor 1 and 2, GAD65, GAD67, GFAP, GLAST, BLBP, TN-C, N-cadherin, Nestin and SOX2.

The separation of the populations that present the phenotype of interest can be performed by means of affinity separation techniques, including, but not being limited to: magnetic separation (using magnetic particles coated with specific antibodies), affinity chromatography, cytotoxic agents bound to monoclonal antibodies or used together with monoclonal antibodies, and "panning" with the antibody associated to a solid support, and by means of other suitable techniques. It is possible to obtain a more accurate separation by means of flow cytometry, a technique which allows separating cell populations in accordance with the stain intensity, together with other parameters such as cell size and cell complexity.

Therefore, a **second aspect** of the invention relates to a method to isolate epithelial cells circulating in peripheral blood, hereinafter second method of the invention, comprising the steps of the first method of the invention, and further comprises the step of isolating the identified cells by means of an affinity separation technique.

In a more preferred embodiment of this aspect of the invention, the affinity separation technique is selected from techniques that use physical properties, such as size and weight, and techniques that use biological properties (such as markers), or the combination thereof.

In a more preferred embodiment of this aspect of the invention, the affinity separation technique is selected from the list consisting of: magnetic separation, affinity chromatography, cytotoxic agents bound to monoclonal antibodies or used together with monoclonal antibodies, *"panning"* with the antibody associated to a solid support, or any combinations thereof.

In another more preferred embodiment of this aspect of the invention, the affinity separation technique is performed by means of magnetic particles (or microspheres) coated with specific antibodies. In other words, the separation is performed by means of positive immunomagnetic selection. More preferably, a plurality of magnetic particles or micromagnetic spheres is used which have:
a) a diameter between 20 nm and 500 nm, or which allows identifying nuclear and intracytoplasmic markers, more preferably of approximately 50 nm,
b) a diameter between 4000 nm and 6000 nm, preferably of approximately 5000 nm, which allows identifying nuclear markers, or
c) a combination of both.

Even more preferably, a combination of magnetic particles or micromagnetic spheres of type (a) and (b) is used.

A **third aspect** of the invention relates to a method for obtaining data useful for the diagnosis, prognosis and classification of individuals who suffer from a disease that presents with tissue destruction and release of epithelial cells, hereinafter third method of the invention, comprising the steps of the first method of the invention, and further comprises quantifying the number of isolated cells of the second method of the invention.

Additionally, the third method of the invention further comprises comparing the number of cells identified with a reference sample.

The term "reference sample" or "reference quantity", as used in the description, relates to the absolute or relative quantity of epithelial cells which allows individuals who suffer from a disease that presents with epithelial cell destruction to be discriminated from those who do not. Preferably it allows discriminating between those who suffer from the disease and healthy individuals.

The suitable reference quantities may be determined by the method of the present invention from a reference sample which may be analysed, for example, simultaneously or consecutively, together with the biological test sample. Thus, for example but without limiting ourselves, the reference sample may be the negative controls, i.e. the quantities detected by the method of the invention in samples of individuals who do not suffer from any of these diseases. In a particular embodiment, in absence of reference samples, the reference quantity is 0.

In the case of the COPD, the reference quantity relates to the absolute or relative quantity of CPCs which allows discriminating between individuals who suffer from COPD and those who do not. Even more preferably, it allows discriminating between
a) individuals without COPD or lung cancer,
b) individuals with COPD,
c) individuals with adenocarcinoma,
d) individuals with squamous carcinoma,
e) individuals with COPD and adenocarcinoma, or
f) individuals with COPD and squamous carcinoma.
g) Individuals with emphysema

In a preferred embodiment, the disease that presents with destruction of epithelial cells is selected from COPD and/or emphysema or any combinations thereof. Even more preferred, the disease is selected from panacinar emphysema due to Alpha-1-Antitrypsin deficiency; centroacinar or centrilobular emphysema, which is that associated to tobacco consumption; congenital bullous emphysema or associated to processes of pulmonary fibrosis, paraseptal or distal acinar emphysema, related to cicatricial processes.

A **fourth aspect** of the invention relates to a method of diagnosis, prognosis and classification of individuals who suffer from a disease that presents with destruction of epithelial cells, hereinafter fourth method of the invention, comprising the steps of the first and third method of the invention, and further comprising classifying the individual in the group of individuals who have greater risk of suffering from a disease that presents with tissue destruction and release of epithelial cells, when the presence of circulating epithelial cells is detected, preferably when the concentration of these circulating epithelial cells is higher than a reference quantity. In a particular embodiment, in the absence of reference samples, the reference quantity is 0.

In a preferred embodiment of this aspect of the invention, the disease that presents with destruction of the pulmonary parenchyma and release of epithelial cells is selected from COPD and/or emphysema, and other pulmonary processes that present with tissue destruction, such as neoplastic, inflammatory or autoimmune diseases or any combinations thereof. In even more preferred form, the disease is selected from panacinar emphysema due to Alpha-1-Antitrypsin deficiency; which is that associated to tobacco consumption; congenital bullous emphysema or associated to processes of pulmonary fibrosis, paraseptal or distal acinar emphysema, related to cicatricial processes.

A **fifth aspect** of the invention relates to a method for monitoring the response to treatment of individuals who suffer from a disease that presents with tissue destruction and release of epithelial cells, hereinafter fourth method of the invention, comprising carrying out the steps of any of the first to third methods of the invention, in a non-simultaneous manner.

In a preferred embodiment of this aspect of the invention, the disease that presents with tissue destruction and release of epithelial cells is selected from COPD and/or emphysema, and other pulmonary processes that present with tissue destruction, such as neoplastic, inflammatory or autoimmune diseases or any combinations thereof. In even more preferred form, the disease is selected from panacinar emphysema due to Alpha-1-Antitrypsin deficiency; which is that associated to tobacco consumption; congenital bullous emphysema or associated to processes of pulmonary fibrosis, paraseptal or distal acinar emphysema, related to cicatricial processes.

The term "monitoring of the response to treatment", as used in the present description, relates to supervision of the development of the disease, such as, for example, but without being limited to, the assessment of the response to a certain treatment of the disease that presents with destruction of epithelial cells, or to a surgical intervention. Therefore, in a preferred embodiment of this aspect of the invention, the monitoring performed posttreatment.

A **sixth aspect** of the invention relates to a method for monitoring the possibility of establishing a prognosis of the disease, in accordance with the absolute or relative quantity of circulating epithelial cells detected, as indicator of amplitude of the direct tissue damage that takes place in each tissue, detected by means of the fourth method of the invention. Therefore, in a preferred embodiment of this aspect of the invention, the establishment of a prognosis of the disease.

### MEDICAL USES OF THE INVENTION

A seventh **aspect** of the invention relates to the use of a pharmaceutical composition comprising an active ingredient which is selected from a β2-agonist, an anticholinergic agent, a compound of the group of corticosteroids, a phosphodiesterase inhibitor and an immune system suppressant, in the preparation of a medicine for the treatment of an individual with COPD and/or emphysema identifiable by the third or fourth method of the invention.

An eighth **aspect** of the invention relates to the use of a pharmaceutical composition comprising an active ingredient which is selected from platinum coordination complexes (cisplatin or carboplatin), gemcitabine, paclitaxel, docetaxel, etoposide, vinorelbine, pemetrexed, gefitinib, erlotinib, bevacizumab, or any combinations thereof, in the preparation of a medicine for the treatment of an individual with adenocarcinoma and squamous carcinoma, associated to COPD and/or emphysema or not, identifiable by the third or fourth method of the invention.

### KIT OR DEVICE FOR DIAGNOSIS

A ninth **aspect** of the invention relates to a kit or device for diagnosis, hereinafter kit or device of the invention, comprising the necessary elements to analyse the quantity of epithelial cells circulating in peripheral blood, and which, comprising a plurality of magnetic particles or micromagnetic spheres coated with specific antigens, are selected from:
a) a group of particles with a diameter of between 20 nm and 500 nm,
b) a group of particles with a diameter of between 4000 nm and 6000 nm, preferably 5000 nm, or
c) a group formed by particles of the two preceding groups.

More preferably, it comprises the means necessary for comparing the quantity of circulating epithelial cells detected with a reference quantity.

Even more preferably, the kit of the present invention comprises the necessary elements for carrying out any of the methods of the present invention.

The kit may further include, with no type of limitation, buffers, agents to prevent contamination, protein degradation inhibitors, etc. Therefore, the kit may include all the supports and receptacles necessary for its implementation and optimization. Preferably, the kit further comprises the instructions for carrying out any of the methods of the invention.

The kit may also be automated, or can be incorporated in devices capable of carrying out the isolation of cell types that are recognised by antibodies that are conjugated to magnetic particles, and using a liquid fluid, preferably using microfluidic techniques.

An example of this type of devices are the IsoFlux systems, developed by Fluxion Biosciences.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following examples and figures are provided for illustrative purposes, and are not intended to limit the scope of the present invention.

### EXAMPLES OF EMBODIMENT OF THE INVENTION

### MATERIALS AND METHODS

### Determination of the specificity and sensitivity of the methodology from cultures of H820 pulmonary cells, as model for the study of CPCs

1. Cell culture: The lung cancer cell lines were obtained from the American Type Culture Collection (ATCC, Manassas, VA, USA) (15). The H820 cells were maintained in RPMI 1640 medium supplemented with 10% of foetal bovine serum and 100 U / ml of penicillin and 100 ng / ml of streptomycin at 37°C in an incubator humidified with 5% of CO2.
2. Antibodies used to detect CPCs:
   - Cytokeratin: pan-cytokeratin (AE1/AE3 clone) (Sigma Aldrich). Cytokeratins are proteins of the cytoskeleton present in both normal and tumor epithelial cells.
   - Anti-CD44v6: Anti-CD44v6 polyclonal antibody produced in rabbits (AB2080) (Merckmillipore). In the respiratory apparatus, expression of CD44v6 can be verified in the normal pulmonary epithelium (preferably type II pneumocytes) and that seems to play an important role in maintenance of tissue architecture.
3. Experiments with total blood: cells of type II pneumocytes were isolated from blood using double gradient centrifugation with Ficoll1, 119 g / ml (Histopaque 1119) and 1.058 g / ml based on 16% W/V Ficoll PM400. Due to the density of the Ficoll 1.058 g / ml and blood, approximately 1.060 g / ml, and to avoid the mixing, the blood must be previously diluted in 19% PBS1X (4 ml of blood / 17.3 ml of PBS1X).

10 ml of Ficoll 1119 were placed in the lower part of a 50 ml conical bottom tube, then 5 ml of Ficoll 1058, the diluted blood was carefully added over the Ficoll double gradient.

The tubes were centrifuged at 700G for 45 minutes without interruption (Allegra X-12R centrifuge (Beckman Coulter), then the mononuclear cell phase was recovered between the two ficoll layers and they were permeabilized and fixed in accordance with the enrichment and detection kit of cell carcinoma with MACS technology (MiltenyiBiotec, BergischGladbach, Germany). Then, these cells with incubated with a specific antibody of multiple Cytokeratins (CK3-11D5) (MiltenyiBiotec, BergischGladbach, Germany) which recognised cytoplasmic cytokeratins 7, 8, 18 and 19 and later with a FITC-anti-cytokeratin antibody (clone: CK3-6H5; MiltenyiBiotec). Finally, the cells positive for cytokeratin were obtained through magnetic separation columns of MACS cells (MiltenyiBiotec) and they were centrifuged on glass sheets coated with poly-L-lysine.

The cells positive for cytokeratin were viewed and were localized under epifluorescent microscope, observing their specific green cytoplasmic signal.

Next, the samples were incubated with an anti-CD44v6 polyclonal antibody, exon v6rabbit (AB2080 Merckmillipore), combined with a secondary antibody Alexa Fluor 633 of Goat anti Rabbit IgG (H + L) (A-21070 ThermoFisher) and mounted using VECTASHIELD with DAPI mounting medium (Vector Labs). Finally, the slides were viewed under Zeiss LSM 710 confocal / multiphoton laser scanning microscope [Ortega, F.G. et al. miRNA in situ hybridization in circulating tumor cells - MishCTC. Sci. Rep. 5, 9207; DOI:10.1038/srep09207 (2015)].
4. Determination of CPCs in healthy non-smoking subjects: after the determination of CPCs in patients with COPD, CPCs were determined with this methodology in 10 healthy subjects without prior respiratory disease nor prior history of tobacco exposure.
5. Determination of sensitivity and specificity of the assay: Determination of CPCs in patients with COPD

### Study design

Cross-sectional observational study performed in outpatient mode of two pneumology services, during the periods of October 2016 to January 2017.

### Study population

The study had the participation of adult patients over the age of 40 with a previous diagnosis of COPD in accordance with the international guidelines (4), defined based on an accumulated consumption of at least 10 pack- year and a post bronchodilation quotient and FEV 1 /FVC< 0.70 and at least a prior monitoring of 3 years in doctor's surgery. Exclusion criteria included the presence of an exacerbation of COPD in the 4 weeks prior to the visit, the precedent of surgical intervention or taking of a biopsy for any reason in the previous month, the presence of an alpha-1 antitrypsin deficiency or other chronic respiratory disease aside from COPD and the existence of a prior neoplasia, and the refusal to participate in the study or incapacity to perform the complementary explorations or answer the questionnaires.

### Study variables

For each patient, information was collected about their sociodemographic details, toxic habits and concomitant diseases. All patients performed a post bronchodilator spirometry according to current guidelines and validated questionnaires about their respiratory disease (dyspnea measured using the mM RC scale, score of the COPD- CAT®, COPD Assessment test), and a multidimensional assessment of the severity of the disease using the BODEx index. Furthermore, for each patient, the annualized drop in lung function was collected. To determine the CPCs, 10 mL of cephalic vein peripheral blood was extracted (after discarding the first 10 mL to avoid contamination by epithelial cells from the epidermis) in EDTA tubes (Vacutainer® EDTA tubes)(BD Biosciences, Franklin Lakes, NJ, USA) and said samples were later processed at ambient temperature in a period less than 2 hours avoiding the exposure of the samples to natural light.

### Statistical analysis

The qualitative variables are expressed as absolute and relative frequencies, the quantitative variables depending on whether they follow a normal distribution or not (after the application of the Kolmogorov-Smirnov or Shapiro-Wilk test) are expressed Md±SD (mean, standard deviation) and range (minimum and maximum) P50 [P25 - P75] (median, interquartile range) respectively. The comparison of the quantitative variables according to the study groups was performed using ANOVA for independent samples or Kruskal Wallis H test (depending on whether or not they follow normal distribution). The level of statistical significance was established at p < 0.05. The statistical analysis was performed with the Statistical Package for Social Sciences (SPSS Inc., Chicago, IL, USA) version 20.0.

### Ethical aspects

The project was approved by the Ethics and Clinical Research Committee of Granada University. The principles of the Declaration of Helsinki were followed for research projects with human beings. All the participants were informed of the nature of the study and its objectives and granted their participation in it by signing the informed consent.

### Assay results

Seventeen patients with COPD were included in the study to determine CPC. The basal characteristics of the patients with COPD who participated in the study are shown in Table 1. In short, they were patients of sixty years of age, largely men, with a high tobacco consumption and more than 25% were current smokers. They had bronchial obstructions of moderate severity and the large majority were included in group B of the Global Obstructive Lung Disease (GOLD) classification (58.8% of the total sample).

**Table 1. Clinical characteristics of patients with COPD included in the study to isolate CPC. The continuous data are shown as the mean ± standard deviation or median (interquartile range) and the categorical variables as n (%). BMI = Body mass index. Post-BD: post bronchodilatory test. CAT = COPD assessment test. mMRC: modified Medical Research Council scale. BODEx index = BMI, obstruction of air flow, dyspnea and severe exacerbations.**

| | **N = 17** |
|---|---|
| **Age (years)** | 68.9 ± 9.5 |
| **Sex M/F (%)** | 15/2 (88.9%/ 11.1%) |
| **BMI, kg/m²** | 27.4 ± 5.0 |
| **Smoking history** | |
| Current smoker n (%) | 5 (27.8%) |
| Packs/years | 46.4 ± 13.3 |
| **Lung function test** | |
| FEV₁, post-BD, L | 1.71 ± 1.01 |
| FEV₁, post-BD % pred | 51.3 ± 24.6 |
| FVC, post-BD % pred | 67.8 ± 17.1 |

| **Severity of the limitation of air flow, n (%)** | |
|---|---|
| Average | 1 (5.6%) |
| Moderate | 8 (47.1%) |
| Severe/very severe | 8 (47.1 %) |
| **CAT scores** | 13.0 ± 7.7 |
| **mMRC dyspnea scale** | 2 (1-3) |

| **History of exacerbation, prev. year** | |
|---|---|
| Moderate exacerbations, prev. year | 0.71 ± 0.7 |
| Severe exacerbations, prev. year | 0.29 ± 0.61 |

| **GOLD 2017 grades, n (%)** | |
|---|---|
| GOLD A | 4 (23.5%) |
| GOLD B | 10 (58.8%) |
| GOLD C | 1 (5.9%) |
| GOLD D | 2 (11.8%) |
| **Severity of disease, BODEx index** | 2 (0-4) |
| **FEV₁ decline, mL/year** | 90.8 ± 24.5 |

In 6 of the patients (35.29% of the total), the presence was demonstrated of CPC in peripheral blood, with a detection rate of 1 CPC / 106 hematopoietic cells. Figure 1 shows a CPC of a patient with COPD. Table 2 shows the characteristics of patients wherein CPC were isolated, and those wherein the CPC were not isolated.

**Table 2. Characteristic of COPD in patients classified according to CPC isolation. The continuous data are shown as the mean ± standard deviation or median (interquartile range) and the categorical variables as n (%).**

| | **CPCs-(n=11)** | **CPCs+ (n=6)** | **p-value** |
|---|---|---|---|
| **Age, years** | 67.6 ± 9.9 | 70.1 ± 9.6 | 0.621 |
| **Sex, M/F** | 11/0 | 4/2 | 0.041 |

| **Smoking history** | | | |
|---|---|---|---|
| Current smokers, n (%) | 4 (36.4%) | 1 (16.7%) | 0.394 |
| Pack-years | 49.4 ± 12.5 | 40.3 ± 14.8 | 0.199 |
| **FEV1, % pred** | 55.5 ± 27.3 | 43.7 ± 18.7 | 0.364 |
| C**AT score** | 10.6 ± 7.5 | 17.3 ± 6.5 | 0.080 |
| **Exacerbations, prev. year** | 0.88 ± 0.9 | 1.2 ± 1.3 | 0.611 |

| **GOLD 2017 grade** | | | 0.090 |
|---|---|---|---|
| **GOLD A-B** | 10 (90.9%) | 4 (66.7%) | |
| **GOLD C-D** | 1 (9.1%) | 2 (33.3%) | |
| **Dyspnea mMRC ≥ 2** | 4 (36.4%) | 5 (83.3%) | 0.084 |
| **BODEx score** | 1.90 ± 1.75 | 3.14 ± 2.73 | 0.028 |
| **BODEx score ≥ 3 points** | 5 (45.4%) | 4 (66.6%) | 0.236 |

Patients with CPC showed a tendency towards a worse lung function, more moderate and severe exacerbations in the previous year, greater intensity of symptoms by the CAT questionnaire and a greater annualized decrease in lung function, although none of these results was statistically significant (Fig. 2). The patients with CPC in peripheral blood showed a greater severity of the diseases assessed using the BODEx multidimensional index (Fig. 3).

### Immunofluoresence staining of non-tumor lung tissue:

Frozen sections were obtained of non-tumor lung tissues in patients with pneumothorax and they were used as specific control for the expression of CD44v6 from the pneumocytes. Briefly, the sections were defrosted and then fixed with 100% cold acetone (-20 ° C) during 10 minutes. They were washed twice with PBS1X 0.5% Tween, blocked with 3% bovine serum albumin for 1 hour and they were incubated with CD44v6-FITC (MCA1730 BioRad) at 4°C during the night. The sections were washed again three times, they were incubated with DAPI for 5 minutes, they were washed with PBS1X and mounted with the Slowfade Antifade kit (S2828 ThermoFisher) for immunofluorescent display.

The negative controls of other epithelial tissues, such as prostate and liver tissues, were also analysed with the same methodology. In these cases, the staining of CD44v6 was not observed.

## Claims

1. A method for identifying non-tumor epithelial cells circulating in peripheral blood of an individual comprising the steps of incubating the peripheral blood sample with the intracytoplasmic, nuclear and/or surface markers in accordance with the circulating epithelial cell to detect, and identifying the cells by means of immunocytochemical, molecular and/or cytogenetic techniques.

2. The method according to the preceding claim further comprising a prior step of subjecting the sample of peripheral blood of an individual to a density gradient separation.

3. The method according to the preceding claim, wherein, after the density gradient separation, a step is performed consisting of collecting the interphase and washing it with saline buffer before incubating the sample with the markers.

4. The method according to any of claims 1 to 3, wherein the epithelial cells are cells from the lungs, the liver, the pancreas and cells from the gastric and intestinal glands, the liver, genital organs, central nervous system or any combinations thereof.

5. The method according to any of claims 1 to 4, wherein the intracytoplasmic markers are selected from the list consisting of: Cytokeratins, Vimentin, SP1 (Surfactant A and B antigen), or any combinations thereof.

6. The method according to any of claims 1 to 5, wherein the nuclear markers are selected from the list consisting of: transcription factors, and preferably they are selected from Snail, Slug, SOX2, or any combinations thereof.

7. The method according to any of claims 1 to 6, wherein the surface markers are selected from the list consisting of: N-cadherin, EpCam, CD44v6, AXL, EGFR, EMA, MUCINA, CD133, Sca1, or any combinations thereof.

8. The method according to any of claims 1-7, wherein the markers are of circulating pulmonary cells, and are selected from the list consisting of:
• Surface markers: CD44v6, Sca1;
• Intracytoplasmic markers: Cytokeratins, SP1;
• Nuclear markers: SOX2;
• any combinations thereof.

9. A method to isolate epithelial cells circulating in peripheral blood, comprising the steps according to any of claims 1 to 8, and further comprising isolating the identified cells by means of an affinity separation technique.

10. The method to isolate epithelial cells circulating in peripheral blood according to the preceding claim wherein the affinity separation technique is selected from techniques that use physical properties, and techniques that use biological properties, or the combination thereof.

11. The method to isolate epithelial cells circulating in peripheral blood according to any of claims 9 or 10, wherein the affinity separation technique is selected from the list consisting of: magnetic separation, affinity chromatography, cytotoxic agents bound to monoclonal antibodies or used together with monoclonal antibodies, "panning" with the antibody associated to a solid support, or any combinations thereof.

12. The method to isolate epithelial cells circulating in peripheral blood according to any of claims 9 to 11, wherein the affinity separation technique is performed by means of magnetic particles coated with specific antibodies.

13. The method to isolate epithelial cells circulating in peripheral blood according to the claim wherein the magnetic particles coated with antibodies are selected from:
a) a group of particles with a diameter of between 20 nm and 500 nm,
b) a group of particles with a diameter of between 4000 nm and 6000 nm, preferably 5000 nm, or
c) a group formed by particles of the two preceding groups.

14. A method for obtaining data useful for the diagnosis, prognosis and classification of individuals who suffer from a disease that presents with tissue destruction and release of epithelial cells comprising the steps according to any of claims 1 to 13, and further comprises the step of quantifying the number of isolated cells.

15. The method for obtaining data useful for the diagnosis, prognosis and classification of individuals who suffer from a disease that presents with tissue destruction and release of epithelial cells according to the preceding claim further comprising comparing the number of cells identified with a reference sample.

16. A method of diagnosis, prognosis and classification of individuals who suffer from a disease that presents with tissue destruction and release of epithelial cells, comprising the steps as described in claims 14 or 15 and further comprises classifying the individual in the group of individuals who have a risk of suffering from a disease that presents with tissue destruction and release of epithelial cells when the presence of epithelial cells is identified in peripheral blood.

17. The method of diagnosis, prognosis and classification of individuals who suffer from a disease that presents with tissue destruction and release of epithelial cells according to the preceding claim, wherein the disease that presents with tissue destruction and release of epithelial cells is pulmonary emphysema, and circulating pulmonary cells are identified in peripheral blood.

18. A method for monitoring the response to treatment of individuals who suffer from a disease that presents with tissue destruction and release of epithelial cells, comprising carrying out the steps of the method according to any of claims 1 to 15, in a non-simultaneous manner, wherein at least one of the steps of quantifying the circulating epithelial cells is performed after administering the treatment to the individual.

19. The use of a pharmaceutical composition comprising an active ingredient which is selected from a β2-agonist, an anticholinergic agent, a compound of the group of corticosteroids, a phosphodiesterase inhibitor and an immune system suppressant, in the preparation of a medicine for the treatment of an individual with pulmonary emphysema identifiable by a method as described in any of claims 17 to 18.

20. The use of a pharmaceutical composition comprising an active ingredient which is selected from platinum coordination complexes (cisplatin or carboplatin), gemcitabine, paclitaxel, docetaxel, etoposide, vinorelbine, pemetrexed, gefitinib, erlotinib, bevacizumab, or any combinations thereof, in the preparation of a medicine for the treatment of an individual with adenocarcinoma and squamous carcinoma, associated to COPD or not, identifiable by a method as described in any of claims 17 to 18.

21. A kit or device for identification, separation and/or diagnosis comprising the necessary elements to analyse the quantity of epithelial cells circulating in peripheral blood, and comprising:
a) magnetic spheres of a diameter of between 20 nm and 500 nm, preferably 50 nm,
b) magnetic spheres of a diameter of between 4000 and 6000 nm, preferably of approximately 5000 nm, or
c) a combination of both.
